# EUROPEAN PATENT SPECIFICATION

(11) **EP 1 605 990 B1**
(45) Date of publication and mention of the grant of the patent: **06.08.2008**
(21) Application number: 04720927.5
(22) Date of filing: 16.03.2004
(51) Int. Cl.: A61M 1/36

(54) **A DEVICE FOR PROTECTING MEDICAL APPARATUS.**
VORRICHTUNG ZUM SCHUTZ MEDIZINISCHER APPARATUREN
DISPOSITIF POUR LA PROTECTION D'APPAREILS A USAGE MEDICAL

(30) Priority: 21.03.2003 IT MO20030079
(43) Date of publication of application: 21.12.2005
(73) Proprietor: Gambro Lundia AB, 22 643 Lund (SE)
(72) Inventor: NERI, Roberto, I-41037 Mirandola (IT)
(74) Representative: Villanova, Massimo
(86) International application number: PCT/IB2004/000763
(87) International publication number: WO 2004/082732

(56) References cited:
- EP-A- 0 536 297
- EP-A- 1 097 725
- DE-A- 3 126 850
- DE-A- 19 816 871
- GB-A- 2 168 263
- US-A- 4 493 693
- US-A- 5 458 586
- US-A1- 2003 040 687
- US-B1- 6 168 653

## Description

### Background of the Invention.

The invention relates to a device for protecting medical apparatus, in particular from contamination by infectious agents.

Specifically, though not exclusively, the invention can be usefully applied to an apparatus for extracorporeal blood treatment, in particular for treatment of renal insufficiency.

In particular, the invention relates to a transduction-protection device comprising a containing hollow body, having an inlet for communication with an extracorporeal circuit, and an outlet for communication with an operator unit of the medical apparatus, for example with a device for measuring the pressure in the extracorporeal circuit. The device of the operation functions as a transducer, which is both able to transmit pressure from the inlet to the outlet allowing for no drop in pressure and also as an aseptic barrier which protects the medical apparatus from infectious agents originating from the patient.

In more detail, the present invention relates to a device comprising a containing hollow body having an inlet comprising a first tubular connector, destined to be connected to a fluid line associated during operation with an extracorporeal fluid transport circuit, and an outlet comprising a second tubular connector, destined for connection with a fluid line associated during operation to an operator unit of a medical apparatus, the operator unit comprising, for example, a pressure gauge; the first and second tubular connectors being in reciprocal gas communication through an internal cavity of the hollow body, which contains a hydrophobic membrane which defines within the cavity an anti-contamination barrier which is gas-permeable and which is arranged transversally between the first and second tubular connectors.

The device enables transmission of the pressure from the extracorporeal fluid transport circuit to the pressure gauge, without significant losses of head, while at the same time protecting the operators, the medical apparatus, which the pressure gauge is part of, and the surrounding environment, from the risk of contamination from pathogens originating from the fluid running in the extracorporeal circuit. The device can further protect the extracorporeal circuit and therefore the patient too, from intrusion of extraneous particles originating from the medical apparatus.

Transduction-protection devices of the above-described type already exist in the prior art, for example in patents US 4,314,480, EP 0 652 018, US 5,500,003, US 6,086,762, US 6,506,237 and EP 1 097 725, and are commonly known as "blood catchers" or "transducer-protectors".

One of the drawbacks of the known devices is their poor security level: the protective function of the membrane can be lost due to micro-lesions in the membrane itself, or imperfections, even microscopic, in the seal around the perimeter edge of the membrane, which edge is sealed and lies between two plastic elements bearing the two tubular connectors, along an ultrasonic plastic welding zone which, apart from solidly joining the plastic elements and the membrane, also guarantees the seal.

A known solution to this drawback consists in predisposing, along the fluid line, two devices in series, in reciprocal fluid communication. Thanks to this solution, even if one of the two devices were to fail, the protective function should be guaranteed by the other device. This solution, however, leads to increases in costs, due to the need to realize two distinct ultrasonic welding processes, one for each device, and also due to the need subsequently to realize a connection to the fluid line of the two devices.

### Summary of the Invention.

A main aim of the present invention is to provide a device, of the above-described type, which is able to overcome the above-mentioned drawbacks inherent in the prior art.

A further aim of the invention is to make available a simple and economical process for manufacturing the device of the invention.

An advantage of the invention is that it provides a simple and economical device which can guarantee a high degree of security against contamination by infectious agents.

A further advantage of the present invention is that it provides a device which is able to transmit, with extreme ease and reliability, the pressure signal coming from the extracorporeal circuit.

A further advantage is that the invention provides a relatively small device.

These aims and advantages and more besides are all attained by the invention, as it is defined in one or more of the appended claims.

According to a characteristic of the invention, the hollow body has an intermediate portion, comprised between the two tubular connectors, made in a single piece and having a central opening for passage of gas, to which two hydrophobic membranes are associated, on opposite sides of the intermediate portion and peripherally sealed. Each of the hydrophobic membranes functions as an aseptic barrier against contamination.

This characteristic guarantees protective effectiveness, thanks to the presence of two aseptic barriers arranged in series one following another, in a device which is constructionally simple and economic and of contained size.

In a characteristic of the invention, the intermediate portion is plate-shaped, with an axial dimension that is smaller than its radial dimensions, having a central opening for passage of gas.

This allows the various parts making up the device to be solidly joined together; i.e the three portions of the body and the two hydrophobic membranes, by a single ultrasonic welding operation.

Further characteristics and advantages of the present invention will better emerge from the detailed description which follows, of at least one embodiment of the invention, illustrated non-exclusively by way of example in the accompanying figures of the drawings.

### Brief Description of the Drawings.

The description that follows will make reference to the figures of the drawings, provided by way of example and therefore non-limiting, in which:
- figure 1 is a partially-sectioned view of a device made according to the present invention;
- figure 2 is a view in vertical elevation of a part of a dialysis apparatus comprising the device of figure 1;

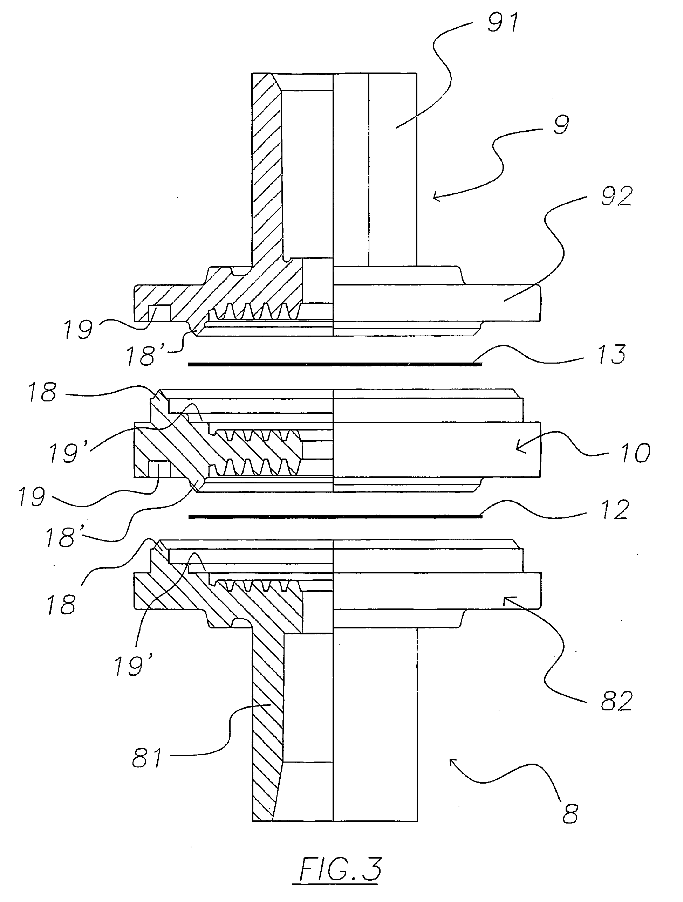

- figure 3 is the device of figure 1 before assembly of the components;
- figure 4 is the device of figure 1 before the ultrasonic welding stage;
- figure 5 is a plan view of a first portion, made of plastic, of the device of figure 1;
- figure 6 is section VI-VI of figure 5;

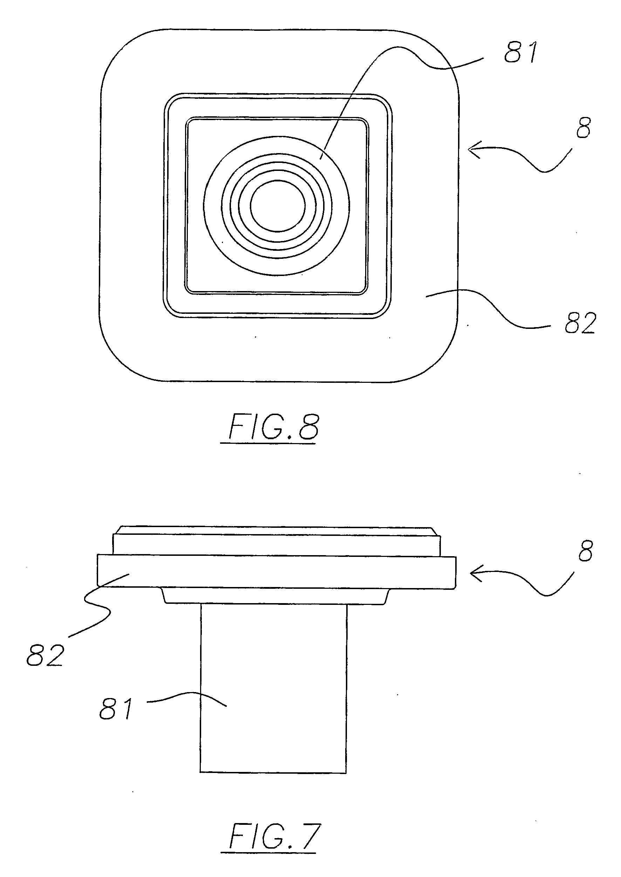

- figure 7 is a side view of the first portion of figure 5;
- figure 8 is a view from below of figure 7;
- figure 9 is a plan view of a second portion, made of plastic, of the device of figure 1;
- figure 10 is section X-X of figure 9;

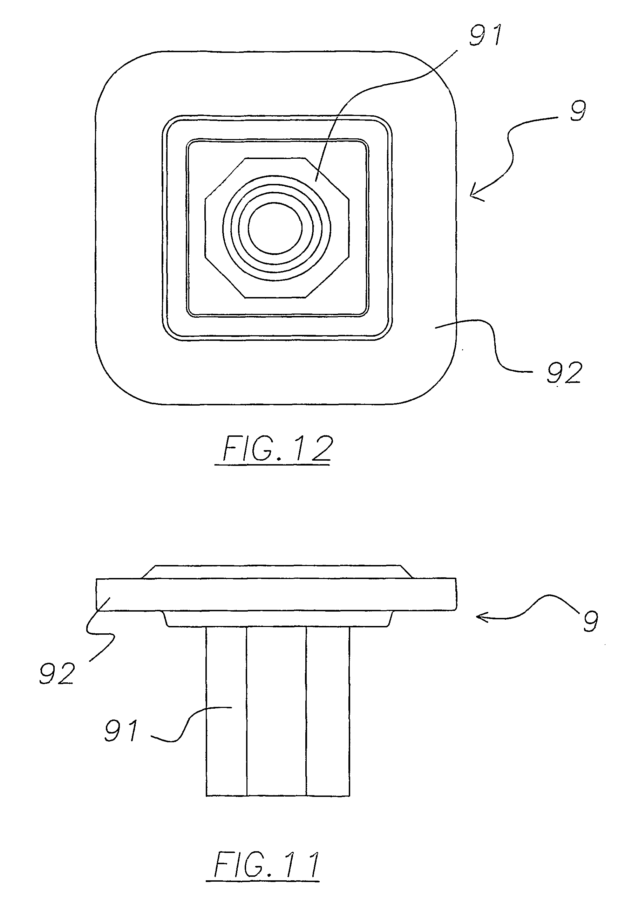

- figure 11 is a side view of the second portion of figure 9;
- figure 12 is a view from below of figure 11;
- figure 13 is a plan view of a third portion, made of plastic, of the device of figure 1;
- figure 14 is section XIV-XIV of figure 13;
- figure 15 is a side view of the third portion of figure 13;
- figure 16 is a view from below of figure 15;
- figure 17 is a plan view of one of the two protective membranes of the device of figure 1;
- figure 18 is a plan view of the membrane of figure 13 applied on the first plastic portion of figure 5;
- figures from 19 to 22 show four embodiments of the device of the invention with various types of tubular connections;
- figure 23 is a partially sectioned view of a further embodiment not forming part of present invention.

### Detailed Description.

1 denotes in its entirety a device for protecting medical apparatus from contamination by infectious agents. The protection device 1 is used in particular in combination with apparatus for extracorporeal blood treatment, for example in treatment of renal insufficiency (dialysis machines).

The device 1 is predisposed to operate along an auxiliary line 2 which connects an extracorporeal blood circuit 3 with a medical apparatus 4. The device 1 has the double function of protecting and transducing, i.e. of protecting the medical apparatus 4, the operators and the surrounding environment, while at the same time transmitting the extracorporeal circuit 3 pressure to a pressure gauge, known and not illustrated, which is part of the medical apparatus 4, with no significant loss of head.

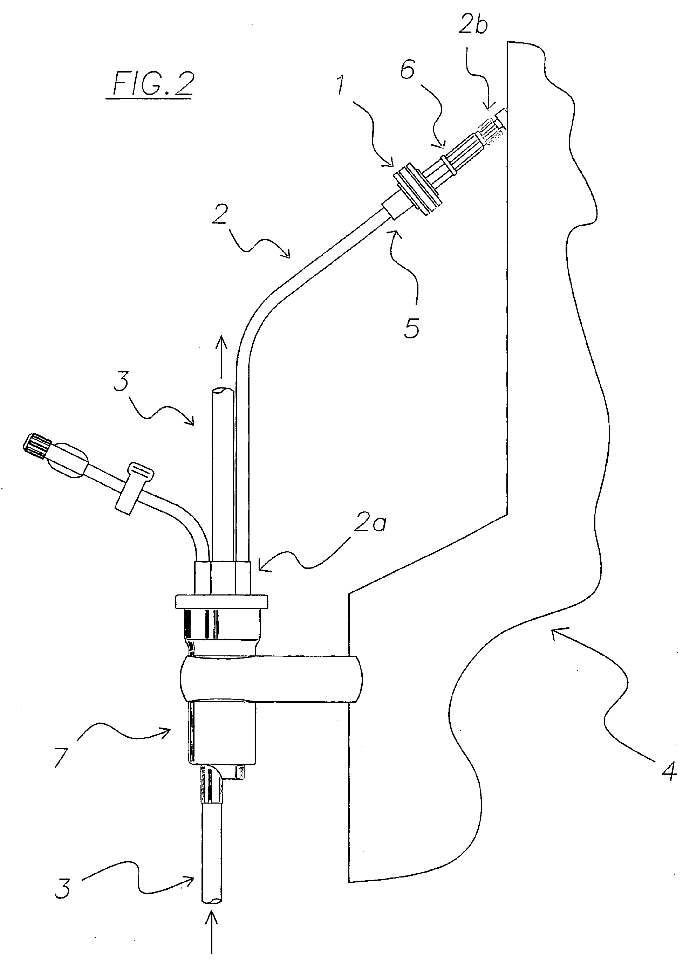

The device 1 comprises a hollow body having an inlet 5 communicating with the extracorporeal blood circuit 3, and an outlet 6, communicating with the medical apparatus 4. The auxiliary line 2, in which the protection device 1 is inserted, has one end, an inlet end 2a, connected to a zone of the extracorporeal circuit 3, and another end, an outlet end 2b, connected to the medical apparatus 4. In more detail, in the embodiment illustrated in figure 2, the inlet end 2a of the auxiliary line 2 is attached to a rigid-walled container 7 (for example an arterial chamber of an extracorporeal blood circuit); the outlet end 2b of the auxiliary line 2 is connected to a pressure transducer (not illustrated) which transforms the pressure signal, transmitted through the protection device 1, into a corresponding electric signal which is received by a control unit of the medical apparatus 4. Figure 2 partially shows a front panel of the medical apparatus 4, which exhibits a seating predisposed for removable connection with an end connector of the auxiliary line 2.

The hollow body comprises a first end portion 8, a second end portion 9 opposite the first end portion 8, and a third intermediate portion 10 interpositioned between the two end portions 8 and 9. Each of the three portions, 8, 9 and 10, is made of a rigid plastic material (in the illustrated embodiment PETG), and is in a single piece and manufactured by plastic moulding.

The first end portion 8 has a first tubular connector 81, which defines the inlet 5 of the hollow body, destined to be connected with a fluid line connected during operation to the extracorporeal fluid transport circuit. In the illustrated embodiment, the fluid line (auxiliary line 2) comprises a flexible tube, made of a plastic material, joined to the first tubular connector 81 by force-fitting and gluing.

The second end portion 9 has a second tubular connector 91, which defines the outlet 6 of the hollow body, and which is in gas communication with the first tubular connector 81 through an internal cavity 11 of the hollow body; the second tubular connector 91 is destined to be connected to a fluid line which during operation is connected to the pressure gauge of the medical apparatus 4. The two tubular connectors 81 and 91 are reciprocally coaxial.

The two end portions 8 and 9 each have a flanged part 82 and 92 which exhibits, as will be better described herein below, at least one permanent join zone with the third intermediate portion 10.

The third intermediate portion 10 is plate-shaped, centrally open, coaxial with the two end portions 8 and 9, and has an axial dimension which is smaller than the radial dimensions thereof.

The central opening of the third intermediate portion 10 allows passage of gas. The third intermediate portion 10 is, in substance, a flanged union element between the two end portions 8 and 9. The axial dimension of the flanged element is about the same size (not more than two or three times bigger) as the axial dimension of the flanged parts 82, 92 of the first and second end portions 8 and 9.

The third intermediate portion 10 is coupled on a first side thereof to the first end portion 8 along an annular union zone 102 and is coupled on a second side thereof, opposite to the first side, to the second end portion 9, along an annular union zone 104. The annular union zones 102 and 104 are permanent coupling zones, and are coaxial one to another and have about the same diameter, and in the illustrated embodiment are constituted by ultrasonic weld zones.

As mentioned above, the third intermediate portion 10 is a press-formed single piece plastic element. The third intermediate portion 10 has a prevalently radially-extending development, rather than axially-extending. In other words, the third intermediate portion 10 is more greatly developed in breadth rather than in length, where for length we intend the direction of the longitudinal axis of the hollow body.

The device comprises a first filter membrane 12, with hydrophobic properties, contained in the hollow body and defining, in the cavity 11 of the hollow body, a first anti-contamination aseptic barrier, arranged transversally between the first end portion 8 and the third intermediate portion 10. A second filter membrane 13 is housed in the hollow body, which second membrane has hydrophobic properties and defines, in the cavity 11 of the hollow body, a second anti-contamination aseptic barrier, arranged transversally between the second end portion 9 and the third intermediate portion 10. 101 and 103 indicate two annular liquid-seal zones, located at two peripheral sealed edges of the filtering membranes 12 and 13. The peripheral sealed edge of the first filter membrane 12 is pressed, at the annular seal zone 101, between the first end portion 8 and the third intermediate portion 10. The peripheral sealed edge of the second filter membrane 13 is pressed, at the annular seal zone 103, between the second end portion 9 and the third intermediate portion 10. The two annular seal zones 101 and 103 are coaxial and have about the same diameter. Further, the annular seal zones, 101 and 103, are coaxial to the annular union zones 102 and 104, and have a smaller diameter than the annular union zones 102 and 104.

Both the first membrane 12 and the second membrane 13 are formed by at least two layers, one an operating filtering layer, made of polyetetrafluoroethylene and facing the inlet 5 of the hollow body, and a support layer, made of non-woven polyester and facing the outlet 6 of the hollow body.

Both the first membrane 12 and the second membrane 13 are filter membranes allowing passage of air, so that changes in pressure on one side of each membrane are transmitted to the opposite side, to which a pressure-sensitive device is connected.

In other words, both the first membrane 12 and the second membrane 13 are gas-permeable to enable transmission of a pressure signal through the protection device 1, from the inlet 5 to the outlet 6, i.e. from the extracorporeal circuit 3 to the pressure gauge of the medical apparatus 4, without determining any relevant loss of head; the pressure measured by the medical apparatus 4 thus corresponds to the effective pressure in the extracorporeal circuit 3.

Further, each filter membrane 12 and 13 blocks non-sterile particles and prevents the passage of contaminating agents from one side to the other of the membrane.

The central part of the first membrane 12 faces the central part of the second membrane 13 in a central gas passage zone internally of the hollow body, comprised between the inlet 5 and the outlet 6 of the hollow body.

The first portion of end 8 has an internal surface which faces and is parallel to the first membrane 12 and delimits the internal cavity 11 of the hollow body. The internal surface is arranged perpendicular to the axis of the hollow body and centrally exhibits an opening for fluid passage. A plurality of reliefs 14 are fashioned from the internal surface, which reliefs 14 define a striker surface for the first membrane 12.

The second end portion 9 has an internal surface, facing and parallel to the second membrane 13 and delimiting the cavity 11 internally of the hollow body. The internal surface is perpendicular to the axis of the hollow body and centrally exhibits an opening for fluid passage. A plurality of reliefs 15 are fashioned from this surface and define a striker surface for the second membrane 13.

The third intermediate portion 10 has, on two opposite sides, two internal surfaces which delimit the internal cavity 11 of the hollow body, each of which is perpendicular to the axis of the hollow body and centrally exhibits an opening for fluid passage. A plurality of reliefs 16 is fashioned from a first internal surface, facing and parallel to the first membrane 12 on an opposite side thereof with respect to the internal surface of the first end portion 8. The plurality of reliefs 16 defines a striker surface for the first membrane 12. A further plurality of reliefs 17 are fashioned from a second internal surface, opposite to the first internal surface and facing and parallel to the second membrane 13, on an opposite side to the internal surface of the second end portion 9. The plurality of reliefs 17 define a striker surface for the second membrane 13.

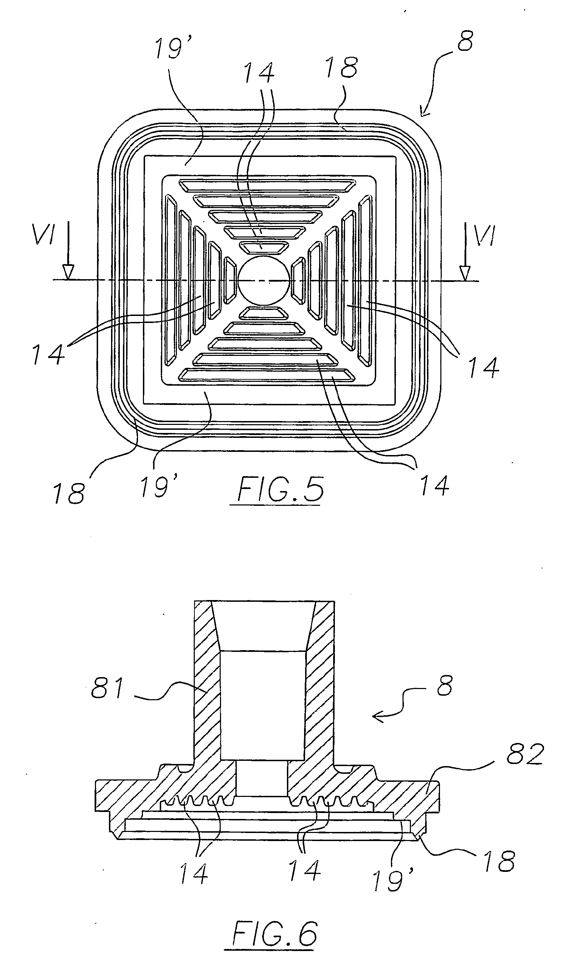

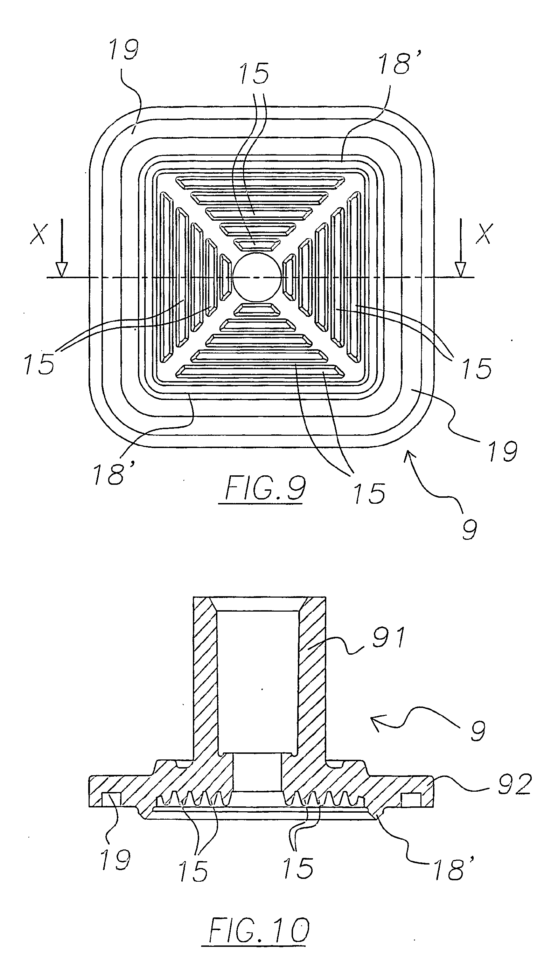

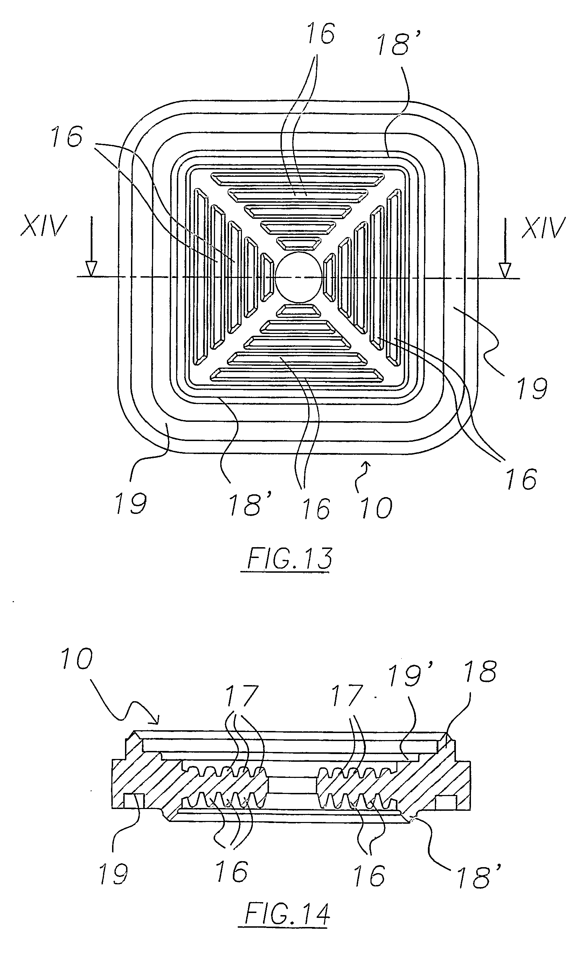

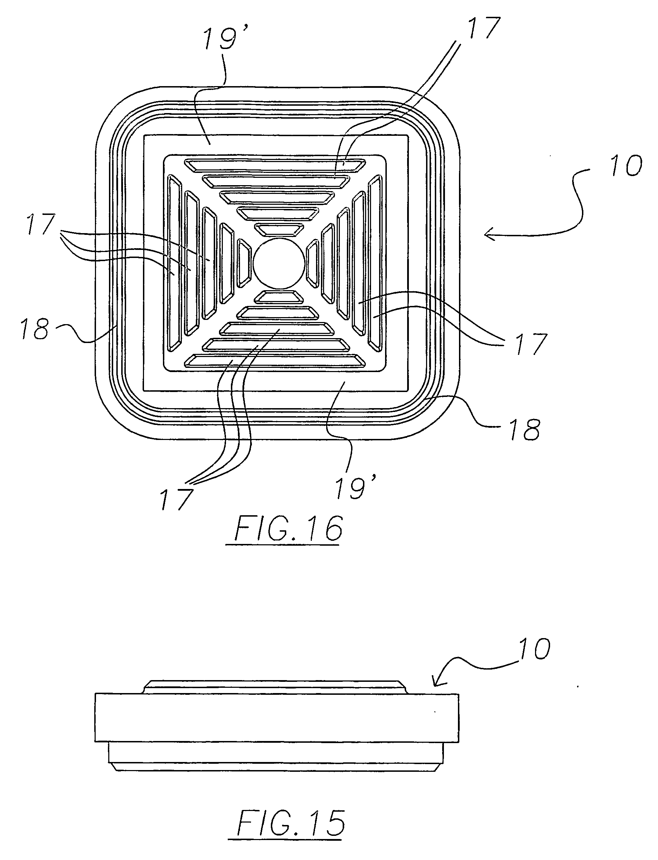

The above-cited pluralities of reliefs 14, 15, 16 and 17, fashioned on each of the three portions 8, 9 and 10 of the hollow body, are fashioned as ribs arranged tangentially, with reference to the longitudinal axis of the hollow body, to define a plurality of tangential channels communicating with a central zone of the cavity 11 of the hollow body, through one or more radial channels defined by the reliefs (as can be seen in figures 5, 9, 13 and 16).

The above-mentioned annular liquid-seal zones 101 and 103, which guarantee the aseptic barrier provided by each membrane 12 and 13, are also zones where the peripheral edges of the membranes 12 and 13, squeezed between the first end portion 8 and the third intermediate portion 10, and the third intermediate portion 10 and the second end portion 9 - are solidly constrained to the hollow body.

The described device is manufactured using a process which includes a preliminary stage of preparation, by means of plastic press-forming, of the three portions 8, 9 and 10 of the hollow body, in which the annular union zones 102 and 104, predisposed for permanent connection by plastic ultrasonic welding, comprise annular welding projections 18 with tapered ends, in particular with triangular meridian sections. The annular welding projections 18, present on one or more of the various portions of the hollow body, are coupled with annular surfaces 19 specially shaped to receive the annular welding projections 18 and situated on another, facing portion of the hollow body.

In more detail, the first end portion 8 exhibits, in the union zone 102, an annular welding projection 18 which corresponds to a flat annular surface 19 lying in an annular recess on the third intermediate portion 10. Further, the first end portion 8 exhibits, in the annular seal zone 101, more internally and having a smaller diameter than the union zone 102, a flat annular surface 19', corresponding with a sealing annular crushing projection 18' located on the third intermediate portion 10. The second end portion 9 exhibits, in the union zone 104, in an annular recess, a flat annular surface 19 corresponding with an annular welding zone 18 located on the third intermediate portion 10; further, the second end portion 9 exhibits, in the seal zone 103, more internal and having a smaller diameter than the union zone 104, a sealing annular crushing projection 18', corresponding with a flat annular surface 19' located on the third intermediate zone 10.

The process also includes a preliminary stage of preparation of the hydrophobic filter membranes 12 and 13, by transversal cutting of a continuous belt of material.

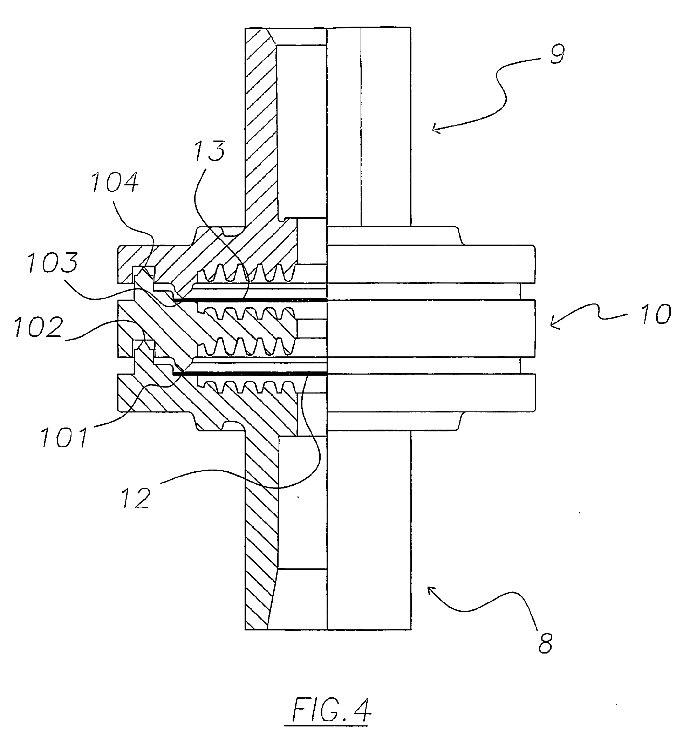

In a subsequent stage, the three portions 8, 9 and 10 and the two filter membranes 12 and 13 are arranged in a group, with the third portion 10 interpositioned between the first and second end portions 8 and 9, the first membrane 12 being arranged transversally between the first and the third portions 8 and 10, and the second membrane 13 being arranged transversally between the third and the second portions 10 and 9, causing the annular welding projections 18 and the sealing crushing projections 18' to contact the corresponding annular surfaces 19 and 19', and interpositioning the perimeter edges of the membranes 12 and 13 between the annular seal zones 101 and 103. This grouped configuration, which precedes the welding stage, is illustrated in figure 4.

The process at this point includes solidly uniting the three portions 8, 9, 10 of the hollow body together, as well as the two hydrophobic protective filter membranes 12 and 13, through a simultaneous solid connection thereof, along the annular union zones 102 and 104. This simultaneous and solid connection comprises, in the illustrated embodiment, a use of ultrasonic energy which, as is known, leads to localized fusion of the annular welding projections 18. During the ultrasonic welding operation compression of the annular union zones is applied, performed with the usual machines for ultrasonic welding. The ultrasonic energy causes a partial fusion, or at least a partial softening, of the material the sealing crushing projections 18' are made of.

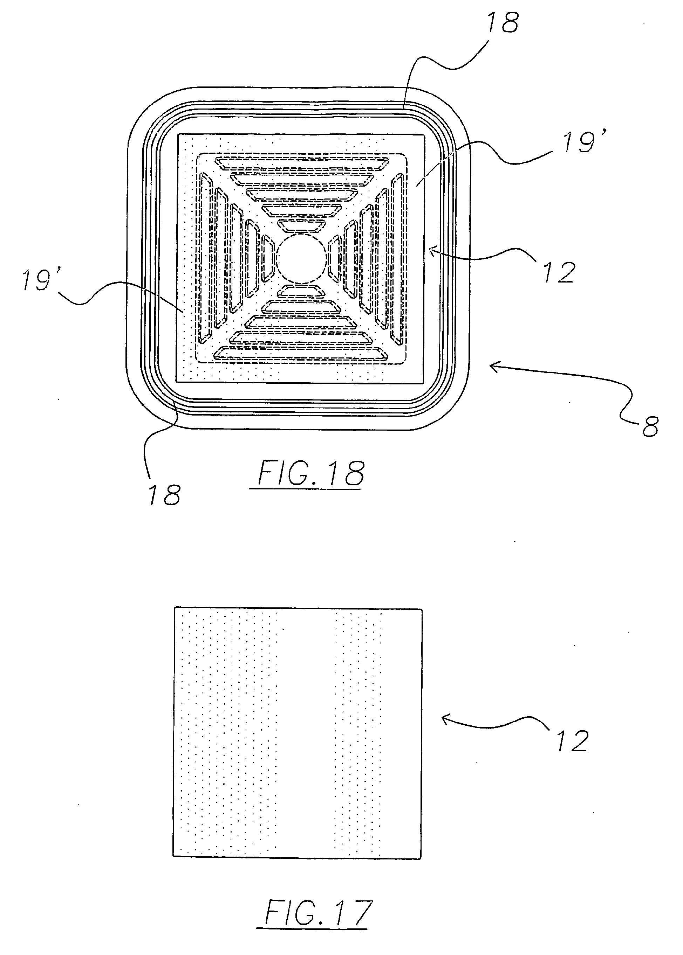

As shown clearly in figures 17 and 18, each membrane 12 and 13 has, observed in plan view, at least one straight perimeter side. In more detail, each membrane 12 and 13 has, seen in plan view, at least a first pair of opposite perimeter sides which are parallel one to another. Each membrane 12 and 13 has, seen in plan view, a second pair of opposite perimeter sides, and in even more detail, each membrane 12 and 13 has, once more seen in plan view, a rectangular shape, which, more specifically, in the illustrated embodiment is square.

At least the two annular seal zones 101 and 103 prevalently follow the shape of the liquid-sealed membrane perimeter zone, and include rounded zones at the corners, to avoid creating union zones with live edges.

The annular union zones 102 and 104, which are arranged coaxially and more peripherally with respect to the annular seal zones 101 and 103, are more or less of the same shape, but wider, as the annular seal zones 101 and 103. In the illustrated embodiment, the annular union zones 102 and 104 have a rectangular shape, in fact are square, and have rounded corners.

In further embodiments which are not illustrated, it would be possible to use membranes having, in plan view, a polygonal shape which is different to what is described above, for example a non-equilateral rectangular shape, or a non-rectangular quadrilateral shape, or a regular polygon with more than four sides, and so on. It would also be possible to use membranes having a shape which is close to a square shape, but having sides which are not straight but rather slightly curved, with non-live but rounded edges.

All the membrane shapes described here have in common the fact of the presence of at least one perimeter side, having a curvature diameter which is greater than the lateral dimension of the membrane, the lateral dimension being considered in a perpendicular direction to the side itself.

Where the perimeter side is straight, the corresponding diameter of curvature can be considered, from a purely mathematical point of view, to be of an infinite length, and thus, from a practical point of view, without doubt longer than the lateral dimension of the membrane.

The square shape, and the shapes described above, which all include at least one straight or nearly straight perimeter side, have, with respect to the usual circular shape, the advantage of offering a greater active membrane surface, in conditions of a same lateral dimension, with a consequent reduction in the drop of pressure and a better transmission of the pressure signal across the device, without losing any efficiency as far as the protective function is concerned. Further, by using membranes with at least one straight or nearly-straight perimeter side, membranes can be produced by a transversal cutting of a continuous tape, with a considerable ensuing reduction, possibly even a complete elimination, of waste of material. A further advantage of the above-described device, with a non-circular peripheral shape, is that the operator can grip the device more easily and handle it more comfortably.

In more general terms, the above-described advantages can be obtained by using a membrane having a plan shape delimited by a determined perimeter border, in which the circle of maximum possible diameter within the border has a total area which is smaller than the area of the membrane; in other words, the membrane is larger than the maximum diameter circle that can be drawn within it.

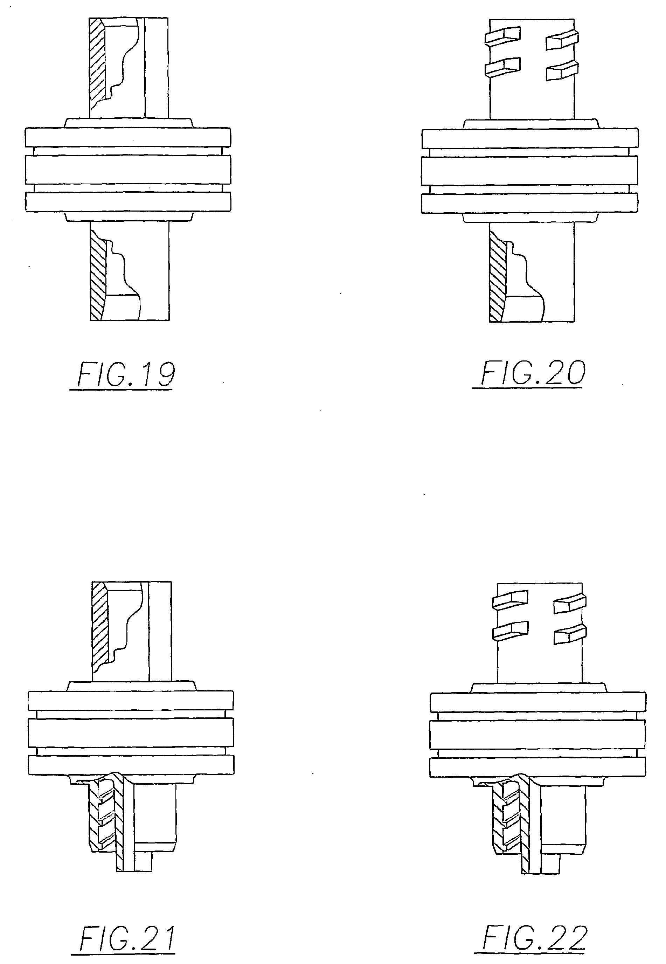

In figures from 19 to 22, four different embodiments of the double-membrane protection device of the invention are illustrated, which differ among themselves in the tubular connections: the first device (figure 19) exhibits two smooth connectors, for coupling to the tubes by force-fitting and gluing; the second device (figure 20) exhibits a smooth connector and a female Luer connector; the third device (figure 21) exhibits a smooth connector and a male Luer connector; the fourth device (figure 22) exhibits a female Luer connector and a male Luer connector.

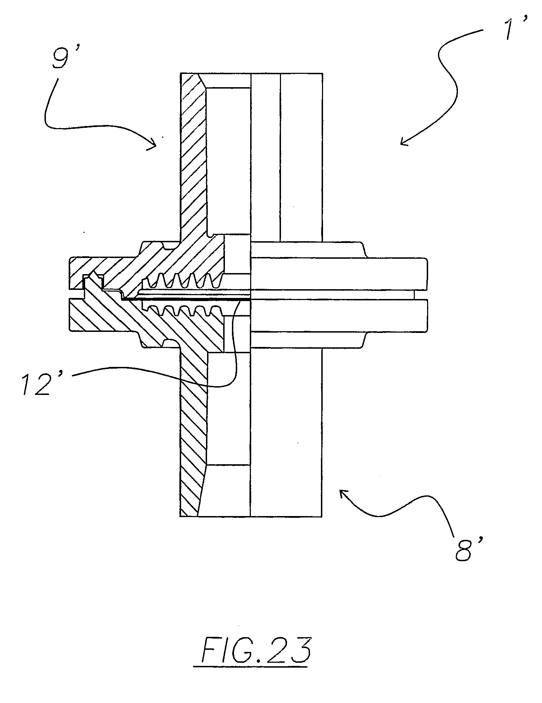

In the embodiment illustrated in figure 23 which does not form part of the present invention, a protection device 1' comprises a single hydrophobic protective membrane 12', having a plan shape which is the same as what is described above, i.e. with one or more straight perimeter sides, or nearly so, or, in more general terms, having a surface areas which is greater than a maximum circle which can be drawn therein. In this case the third intermediate portion is absent, and the only protective membrane 12' is arranged transversally between the first and the second end portions 8' and 9'.

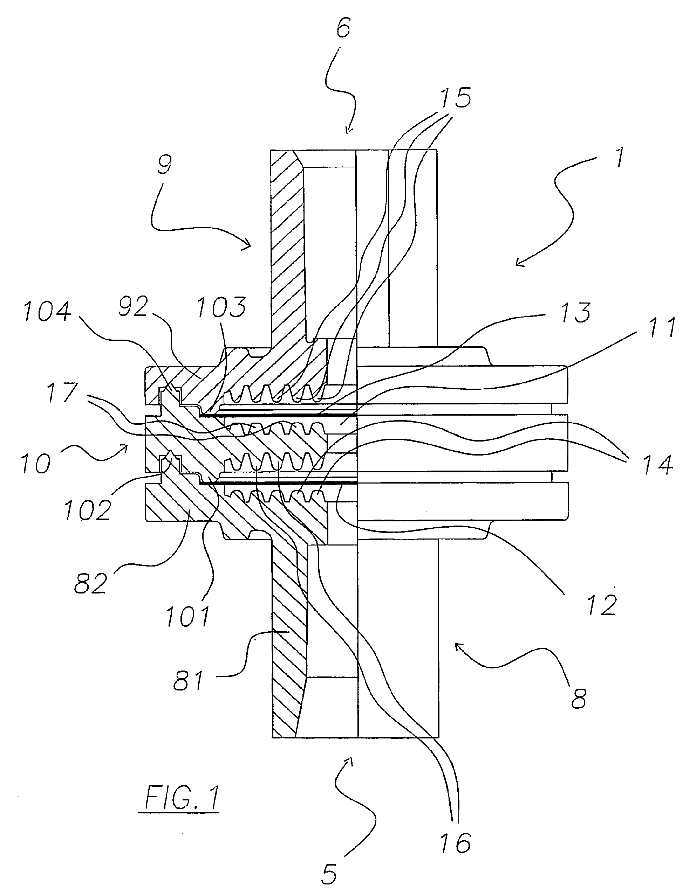

In the first-described and illustrated device in figures from 1 to 18, the two end portions 8 and 9 are already set up and structured to be directly couplable to each other, with the interpositioning of a single filter membrane. Therefore, in figure 1' of figure 23, the two portions 8' and 9' of the hollow body are identical to the two end portions 8 and 9 or the device 1 of figures from 1 to 18, and the filter membrane 12' is identical to the filter membrane 12 or 13.

The device 1' of figure 23, having a single membrane, has the advantage of providing efficient transmission of the pressure, in terms of parity of lateral dimensions, and easy grip and handling.

## Claims

1. A transduction-protection device for protecting medical apparatus, comprising:
a hollow body having at least three portions, in which:
a first end portion (8) has a first tubular connector (81), destined to be connected with a fluid line which fluid line is during operation of the device connected to an extracorporeal circuit (3) for transport of fluid;
a second end portion (9), opposite the first end portion (8) has a second tubular connector (91), in fluid communication with said first tubular connector (81) by means of a cavity (11) which is internal of said hollow body, and destined to be connected to a fluid line which fluid line is during operation of the device connected to a medical apparatus (4); and
a third intermediate portion (10), interpositioned between the first end portion (8) and the second end portion (9);
at least two filter membranes in which:
a first membrane (12), contained in said hollow body, defines, in said cavity (11), a first gas-permeable anti-contamination barrier, arranged transversally between said first end portion (8) and said third intermediate portion (10);
a second membrane (13), contained in said hollow body, defines, in said cavity (11), a second gas-permeable anti-contamination barrier, arranged transversally between said second end portion (8) and said third intermediate portion (10);
said third intermediate portion (10) being coupled at a first side thereof to said first end portion (8) along at least a first union zone (102), and which third intermediate portion (10) is coupled at a second side thereof, opposite to the first side thereof, to said second end portion (9) along at least a second union zone (104);
said first union zone (102) and said second union zone (104) being permanent coupling zones and being annular;
said transduction-protection device comprising at least a first annular seal zone (101) and a second annular seal zone (103), located at a perimeter edge of the first membrane (12) and, respectively, at a perimeter edge of the second membrane (13).

2. The device of claim 1, wherein:
said third intermediate portion (10) has, on two opposite sides, two internal surfaces which delimit said internal cavity (11) of said hollow body, each of which internal surfaces is perpendicular to an axis of the hollow body and centrally exhibits an opening for fluid passage;
a first of said two internal surfaces is facing and parallel to said first membrane (12) on an opposite side thereof with respect to an internal surface of said first end portion (8); and
a second of said two internal surfaces, opposite to said first internal surface, is facing and parallel to said second membrane (13), on an opposite side to an internal surface of said second end portion (9).

3. The device of claim 1 or 2, wherein:
said third intermediate portion (10) exhibits two internal surfaces which delimit said cavity (11);
a plurality of reliefs (16) emerges from a first of said two internal surfaces, which first internal surface faces said first membrane (12), which plurality of reliefs (16) defines a striker surface for said first membrane (12);
a plurality of reliefs (17) emerges from a second of said two internal surfaces, which second internal surface faces the second membrane (13), which plurality of reliefs (17) defines a striker surface for said second membrane (13).

4. The device of any one of the preceding claims, wherein:
said first end portion (8) exhibits an internal surface, which delimits said cavity (11) and which faces said first membrane (12), from which internal surface a plurality of reliefs (14) emerges, defining a striker surface for said first membrane (12);
said second end portion (9) exhibits an internal surface, which delimits said cavity (11) and which faces said second membrane (13), from which internal surface a plurality of reliefs (15) emerges, defining a striker surface for said second membrane (13).

5. The device of claim 3 or 4, wherein said pluralities of reliefs (14; 15; 16; 17) are ribs arranged tangentially, with reference to a longitudinal axis of the hollow body, defining a plurality of tangential channels, communicating with a central zone of said cavity, by means of one or more radial channels defined by said pluralities of reliefs (14; 15; 16; 17).

6. The device of any one of the preceding claims, wherein the central part of said first membrane (12) faces the central part of said second membrane (13) in a central gas passage zone internally of said hollow body, comprised between the inlet (5) and the outlet (6) of the hollow body.

7. The device of any one of the preceding claims, wherein said third intermediate portion (10) is plate-shaped and has a central opening.

8. The device of claim 7, wherein said third intermediate portion (10) is coaxial with said two end portions (8; 9), and has an axial dimension which is smaller than a radial dimensions thereof.

9. The device of any one of the preceding claims, wherein said third intermediate portion (10) is made in a single piece of a rigid material.

10. The device of any one of the preceding claims, wherein said first annular union zone (102) and said second annular union zone (104) exhibit radial dimensions of about a same size and of a greater size than radial dimensions of the first annular seal zone (101) and a second annular seal zone (103).

11. The device of any one of the preceding claims, wherein said first membrane (12) and said second membrane (13) each exhibit at least one straight perimeter side.

12. The device of claim 11, wherein said first membrane (12) and said second membrane (13) each exhibit at least a first pair of perimeter sides which are opposite and parallel to one another.

13. The device of claim 12, wherein said first membrane (12) and said second membrane (13) each exhibit at least a second pair of perimeter sides which are opposite and parallel to one another.

14. The device of claim 13, wherein said first membrane (12) and said second membrane (13) each exhibit a rectangular shape.

15. The device of any one of the preceding claims, wherein said first annular seal zone (101) and said second annular seal zone (103) each exhibit at least two joined adjacent sides which exhibit a rounded corner.

16. The device of claim 15, wherein said first annular seal zone (101) and said second annular seal zone (103) each exhibit a rectangular shape, with rounded corners.

## Patentansprüche

1. Übertragungsschutzvorrichtung zum Schutz von medizinischen Geräten, umfassend:
einen Hohlkörper mit mindestens drei Abschnitten, worin:
ein erster Endabschnitt (8) einen ersten röhrenförmigen Verbinder (81) aufweist, der zur Verbindung mit einer Fluidleitung vorgesehen ist, welche Fluidleitung beim Betrieb der Vorrichtung mit einem extrakorporalen Kreislauf (3) zum Fluidtransport verbunden ist;
ein zweiter, zum ersten Endabschnitt (8) entgegengesetzter Endabschnitt (9) einen zweiten röhrenförmigen Verbinder (91) aufweist, der in Fluidkommunikation mit dem benannten ersten röhrenförmigen Verbinder (81) durch eine, im benannten Hohlkörper liegende Ausnehmung (11) steht und zur Verbindung mit einer Fluidleitung vorgesehen ist, welche Fluidleitung beim Betrieb der Vorrichtung mit einem medizinischen Gerät (4) verbunden ist; und
ein dritter Zwischenabschnitt (10) zwischen dem ersten Endabschnitt (8) und dem zweiten Endabschnitt (9) liegt;
mindestens zwei Filtermembranen, worin:
eine erste Membran (12), die im benannten Hohlkörper aufgenommen ist, in der benannten Ausnehmung (11) eine erste gasdurchlässige Antikontaminationssperre definiert, die quer zwischen dem benannten ersten Endabschnitt (8) und dem benannten dritten Zwischenabschnitt (10) angeordnet ist;
eine zweite Membran (13), die im benannten Hohlkörper aufgenommen ist, in der benannten Ausnehmung (11) eine zweite gasdurchlässige Antikontaminationssperre definiert, die quer zwischen dem benannten zweiten Endabschnitt (9) und dem benannten dritten Zwischenabschnitt (10) angeordnet ist;
wobei der benannte dritte Zwischenabschnitt (10) an einer ersten Seite davon mit dem benannten ersten Endabschnitt (8) entlang mindestens einem ersten Verbindungsbereich (102) gekoppelt ist, und der dritte Zwischenabschnitt (10) an einer zweiten Seite davon, entgegengesetzt zur ersten Seite davon, mit dem benannten zweiten Endabschnitt (9) entlang mindestens einem zweiten Verbindungsbereich (104) gekoppelt ist;
wobei der benannte erste Verbindungsbereich (102) und der benannte zweite Verbindungsbereich (104) ringförmige Dauerkopplungsbereiche sind;
wobei die benannte Übertragungsschutzvorrichtung mindestens einen ersten ringförmigen Dichtbereich (101) und einen zweiten ringförmigen Dichtbereich (103) umfasst, die jeweils an einem Umfangsrand der ersten Membran (12) bzw. an einem Umfangsrand der zweiten Membran (13) liegen.

2. Vorrichtung nach Anspruch 1, worin:
der benannte dritte Zwischenabschnitt (10) an zwei entgegengesetzten Seiten zwei Innenflächen aufweist, die die benannte Innenausnehmung (11) des benannten Hohlkörpers begrenzen, wobei jede der Innenflächen senkrecht zu einer Achse des Hohlkörpers ist und eine Öffnung zum Fluiddurchgang zentral aufweist;
eine erste der benannten beiden Innenflächen zur benannten ersten Membran (12) an einer Seite davon zugewandt und parallel ist, die zu einer Innenfläche des benannten ersten Endabschnittes (8) entgegengesetzt ist; und
eine zweite der benannten beiden Innenflächen zur benannten zweiten Membran (13) an einer Seiten davon zugewandt und parallel ist, die zu einer Innenfläche des benannten zweiten Endabschnittes (9) entgegengesetzt ist.

3. Vorrichtung nach Anspruch 1 oder 2, worin:
der benannte dritte Zwischenabschnitt (10) zwei Innenflächen aufweist, die die benannte Ausnehmung (11) begrenzen;
eine Vielzahl von Vorsprüngen (16) aus einer ersten der benannten beiden Innenflächen ragt, welche erste Innenfläche zur benannten ersten Membran (12) zugewandt ist, welche Vielzahl von Vorsprüngen (16) eine Anschlagsfläche für die benannte erste Membran (12) definiert;
eine Vielzahl von Vorsprüngen (17) aus einer zweiten der benannten beiden Innenflächen ragt, welche zweite Innenfläche zur benannten zweiten Membran (13) zugewandt ist, welche Vielzahl von Vorsprüngen (17) eine Anschlagsfläche für die benannte zweite Membran (13) definiert.

4. Vorrichtung nach irgendeinem der vorherigen Ansprüche, worin:
der benannte erste Endabschnitt (8) eine Innenfläche aufweist, die die benannte Ausnehmung (11) begrenzt und zur benannten ersten Membran (12) zugewandt ist, aus welcher Innenfläche eine Vielzahl von Vorsprüngen (14) ragt, die eine Anschlagsfläche für die benannte erste Membran (12) definiert;
der benannte zweite Endabschnitt (9) eine Innenfläche aufweist, die die benannte Ausnehmung (11) begrenzt und zur benannten zweiten Membran (13) zugewandt ist, aus welcher Innenfläche eine Vielzahl von Vorsprüngen (15) ragt, die eine Anschlagsfläche für die benannte zweite Membran (13) definiert.

5. Vorrichtung nach Anspruch 3 oder 4, worin die benannten Vielzahlen von Vorsprüngen (14; 15; 16; 17) Rippen sind, die tangential zu einer Längsachse des Hohlkörpers angeordnet sind und eine Vielzahl von Tangentialkanälen definieren, die mit einem Zentralbereich der benannten Ausnehmung durch einen oder mehrere, von den benannten Vielzahlen von Vorsprüngen (14; 15; 16; 17) definierte Radialkanäle in Kommunikation stehen.

6. Vorrichtung nach irgendeinem der vorherigen Ansprüche, worin der Zentralteil der benannten ersten Membran (12) zum Zentralteil der benannten zweiten Membran (13) in einem Zentralgasdurchgangsbereich innerhalb von dem benannten Hohlkörper zugewandt ist, welcher Zentralgasdurchgangsbereich zwischen dem Eingang (5) und dem Ausgang (6) des Hohlkörpers eingeschlossen ist.

7. Vorrichtung nach irgendeinem der vorherigen Ansprüche, worin der benannte dritte Zwischenabschnitt (10) plattenförmig ist und eine zentrale Öffnung besitzt.

8. Vorrichtung nach Anspruch 7, worin der benannte dritte Zwischenabschnitt (10) koaxial zu den benannten beiden Endabschnitten (8; 9) ist und ein axiales Maß besitzt, das kleiner als ein radiales Maß davon ist.

9. Vorrichtung nach irgendeinem der vorherigen Ansprüche, worin der benannte dritte Zwischenabschnitt (10) einstückig aus steifem Material hergestellt ist.

10. Vorrichtung nach irgendeinem der vorherigen Ansprüche, worin der erste ringförmige Verbindungsbereich (102) und der zweite ringförmige Verbindungsbereich (104) radiale Maße besitzen, die jeweils ungefähr so groß wie bzw. größer sind als die radiale Maße des ersten ringförmigen Dichtbereich (101) und eines zweiten ringförmigen Dichtbereich (103).

11. Vorrichtung nach irgendeinem der vorherigen Ansprüche, worin der benannte erste Membran (12) und der benannte zweite Membran (13) mindestens eine geradlinige Umfangsseite besitzen.

12. Vorrichtung nach Anspruch 11, worin die benannte erste Membran (12) und die benannte zweite Membran (13) mindestens ein erstes Paar von Umfangsseiten besitzen, die zueinander entgegengesetzt und parallel sind.

13. Vorrichtung nach Anspruch 12, worin die benannte erste Membran (12) und die benannte zweite Membran (13) mindestens ein zweites Paar von Umfangsseiten besitzen, die zueinander entgegengesetzt und parallel sind.

14. Vorrichtung nach Anspruch 13, worin die benannte erste Membran (12) und die benannte zweite Membran (13) eine rechteckige Form besitzen.

15. Vorrichtung nach irgendeinem der vorherigen Ansprüche, worin der benannte erste ringförmige Dichtbereich (101) und der benannte zweite ringförmige Dichtbereich (103) mindestens zwei verbundene, anliegende Seiten mit einer runden Kante besitzen.

16. Vorrichtung nach Anspruch 15, worin der benannte erste ringförmige Dichtbereich (101) und der benannte zweite ringförmige Dichtbereich (103) eine rechteckige Form mit gerundeten Kanten besitzen.

## Revendications

1. Dispositif de protection de transduction pour protéger des appareils médicaux, comprenant:
un corps creux ayant au moins trois portions, où
une première portion d'extrémité (8) présente un premier connecteur tubulaire (81) apte à être relié à une ligne de fluide, laquelle ligne de fluide est reliée pendant le fonctionnement du dispositif à un circuit extracorporel (3) pour le transport de fluide;
une deuxième portion d'extrémité (9), opposée à la première portion d'extrémité (8), présente un deuxième connecteur tubulaire (91) en communication de fluide avec ledit premier connecteur tubulaire (81) au moyen d'une cavité (11) logée à l'intérieur dudit corps creux, et apte à être relié à une ligne de fluide, laquelle ligne de fluide est reliée pendant le fonctionnement du dispositif à un appareil médical (4); et
une troisième portion intermédiaire (10) qui est placée entre la première portion d'extrémité (8) et la deuxième portion d'extrémité (9);
au moins deux membranes de filtration où:
une première membrane (12) contenue dans ledit corps creux définit dans ladite cavité (11) une première barrière anticontamination perméable aux gaz, rangée transversalement entre ladite première portion d'extrémité (8) et ladite troisième portion intermédiaire (10);
une deuxième membrane (13) contenue dans ledit corps creux définit dans ladite cavité (11) une deuxième barrière anticontamination perméable aux gaz, rangée transversalement entre ladite deuxième portion d'extrémité (8) et ladite troisième portion intermédiaire (10);
ladite troisième portion intermédiaire (10) étant couplée sur un premier côté de celle-ci avec ladite première portion d'extrémité (8) le long d'au moins une première zone de jonction (102), et ladite troisième portion intermédiaire (10) étant couplée sur un deuxième côté de celle-ci, opposé au premier côté de celle-ci, avec ladite deuxième portion d'extrémité (9) le long d'au moins une deuxième zone de jonction (104);
ladite première zone de jonction (102) et ladite deuxième zone de jonction (104) étant des zones d'accouplement permanent annulaires;
ledit dispositif de protection de transduction comprenant au moins une première zone d'étanchéité annulaire (101) et une deuxième zone d'étanchéité annulaire (103), respectivement placées sur un bord périphérique de la première membrane (12) et sur un bord périphérique de la deuxième membrane (13).

2. Dispositif selon la revendication 1, où:
ladite troisième portion intermédiaire (10) présente sur deux côtés opposés deux surfaces intérieures délimitant ladite cavité intérieure (11) dudit corps creux, chacune des surfaces intérieures étant perpendiculaire à un axe du corps creux et présentant en position centrale une ouverture de passage de fluide;
une première desdites deux surfaces intérieures est en face de et parallèle à ladite première membrane (12) sur un côté opposé de celle-ci par rapport à une surface intérieure de ladite première portion d'extrémité (8); et
une deuxième desdites deux surfaces intérieures, opposée à ladite première surface intérieure, est en face de et parallèle à ladite deuxième membrane (13) sur un côté opposé de celle-ci par rapport à une surface intérieure de ladite deuxième portion d'extrémité (9).

3. Dispositif selon la revendication 1 ou 2, où:
ladite troisième portion intermédiaire (10) présente deux surfaces intérieures délimitant ladite cavité (11);
une pluralité de reliefs (16) émerge d'une première desdites deux surfaces intérieures, laquelle première surface intérieure est en face de ladite première membrane (12), laquelle pluralité de reliefs (16) définit une surface de butée pour ladite première membrane (12);
une pluralité de reliefs (17) émerge d'une deuxième desdites deux surfaces intérieures, laquelle deuxième surface intérieure est en face de ladite deuxième membrane (13), laquelle pluralité de reliefs (17) définit une surface de butée pour ladite deuxième membrane (13).

4. Dispositif selon une quelconque des revendications précédentes, où:
ladite première portion d'extrémité (8) présente une surface intérieure qui délimite ladite cavité (11) et qui est en face de ladite première membrane (12), de laquelle surface intérieure émerge une pluralité de reliefs (14) définissant une surface de butée pour ladite première membrane (12);
ladite deuxième portion d'extrémité (9) présente une surface intérieure qui délimite ladite cavité (11) et qui est en face de ladite deuxième membrane (13), de laquelle surface intérieure émerge une pluralité de reliefs (15) définissant une surface de butée pour ladite deuxième membrane (13).

5. Dispositif selon la revendication 3 ou 4, où lesdites pluralités de reliefs (14; 15; 16; 17) sont des nervures rangées tangentiellement par rapport à un axe longitudinal du corps creux, définissant une pluralité de canaux tangentiels qui communiquent avec une zone centrale de ladite cavité, au moyen d'une ou plusieurs canaux radiaux définis par lesdites pluralités de reliefs (14; 15; 16; 17).

6. Dispositif selon une quelconque des revendications précédentes, où la partie centrale de ladite première membrane (12) est en face de la partie centrale de ladite deuxième membrane (13) dans une zone centrale de passage de gaz logée à l'intérieur dudit corps creux, comprise entre l'entrée (5) et la sortie (6) du corps creux.

7. Dispositif selon une quelconque des revendications précédentes, où ladite troisième portion intermédiaire (10) a la forme d'une plaque et présente une ouverture centrale.

8. Dispositif selon la revendication 7, où ladite troisième portion intermédiaire (10) est coaxiale auxdites deux portions d'extrémité (8; 9) et présente une dimension axial qui est inférieure à une dimension radiale de celles-ci.

9. Dispositif selon une quelconque des revendications précédentes, où ladite troisième portion intermédiaire (10) est réalisée d'une seule pièce dans un matériau rigide.

10. Dispositif selon une quelconque des revendications précédentes, où ladite première zone de jonction annulaire (102) et ladite deuxième zone de jonction annulaire (104) ont des dimensions radiales qui sont respectivement environ les mêmes et supérieures par rapport aux dimensions radiales de la première zone d'étanchéité annulaire (101) et d'une deuxième zone d'étanchéité annulaire (103).

11. Dispositif selon une quelconque des revendications précédentes, où ladite première membrane (12) et ladite deuxième membrane (13) ont chacune au moins un côté périphérique droit.

12. Dispositif selon la revendication 11, où ladite première membrane (12) et ladite deuxième membrane (13) ont chacune au moins une première paire de côtés périphériques opposés et parallèles l'un à l'autre.

13. Dispositif selon la revendication 12, où ladite première membrane (12) et ladite deuxième membrane (13) ont chacune au moins une deuxième paire de côtés périphériques opposés et parallèles l'un à l'autre.

14. Dispositif selon la revendication 13, où ladite première membrane (12) et ladite deuxième membrane (13) ont chacune une forme rectangulaire.

15. Dispositif selon une quelconque des revendications précédentes, où ladite première zone d'étanchéité annulaire (101) et ladite deuxième zone d'étanchéité annulaire (103) ont chacune au moins deux côtés adjacents joints ayant un coin arrondi.

16. Dispositif selon la revendication 15, où ladite première zone d'étanchéité annulaire (101) et ladite deuxième zone d'étanchéité annulaire (103) ont chacune une forme rectangulaire avec coins arrondis.
